(19) Europäisches Patentamt

European Patent Office

Office européen des brevets

(11) **EP 1 346 216 B1**

(12) **EUROPÄISCHE PATENTSCHRIFT**

(45) Veröffentlichungstag und Bekanntmachung des Hinweises auf die Patenterteilung:
**05.10.2005 Patentblatt 2005/40**

(51) Int Cl.⁷: **G01N 33/483**, G01R 33/465, B01D 59/44

(21) Anmeldenummer: **01984872.0**

(22) Anmeldetag: **19.12.2001**

(86) Internationale Anmeldenummer:
**PCT/EP2001/015071**

(87) Internationale Veröffentlichungsnummer:
**WO 2002/050560 (27.06.2002 Gazette 2002/26)**

(54) **VERFAHREN ZUR BESTIMMUNG DER HERKUNFT UND AUTHENTIZITÄT BIOLOGISCH, BIOCHEMISCH UND ERNÄHRUNGSPHYSIOLOGISCH RELEVANTER STOFFE DURCH MESSUNG IHRER POSITIONELLEN SAUERSTOFF- BZW. WASSERSTOFF-ISOTOPEN-VERHÄLTNISSE**

METHOD FOR DETERMINATION OF THE ORIGIN AND AUTHENTICITY OF MATERIALS WITH BIOLOGICAL BIOCHEMICAL AND NUTRITIONAL PHYSIOLOGICAL RELEVANCE

PROCEDE POUR DETERMINER L'ORIGINE ET L'AUTHENTICITE DE PRODUITS PERTINENTS DU POINT DE VUE BIOLOGIQUE, BIOCHIMIQUE ET PHYSIOLOGIQUE NUTRITIONNEL

(84) Benannte Vertragsstaaten:
AT BE CH CY DE DK ES FI FR GB GR IE IT LI LU MC NL PT SE TR

(30) Priorität: 21.12.2000 DE 10064457
26.07.2001 DE 10136460

(43) Veröffentlichungstag der Anmeldung:
24.09.2003 Patentblatt 2003/39

(73) Patentinhaber: **Schmidt, Hanns-Ludwig**
**84036 Landshut (DE)**

(72) Erfinder:
• **SCHMIDT, Hanns-Ludwig**
**84036 Landshut (DE)**
• **FUGANTI, Claudio, E.P. Sintesi srl**
**I-20129 Milano (IT)**

(74) Vertreter: **Rapp, Bertram et al**
**Charrier Rapp & Liebau**
**Patentanwälte**
**Postfach 31 02 60**
**86063 Augsburg (DE)**

(56) Entgegenhaltungen:
• **HAGEDORN M L: "DIFFERENTIATION OF NATURAL AND SYNTHETIC BENZALDEHYDES BY DEUTERIUM NUCLEAR MAGNETIC RESONANCE" JOURNAL OF AGRICULTURAL AND FOOD CHEMISTRY, Bd. 40, Nr. 4, 1992, Seiten 634-637, XP002215564 ISSN: 0021-8561**
• **ZHANG BEN-LI ET AL: "Site-specific hydrogen isotope fractionation in the biosynthesis of glycerol." BIOORGANIC CHEMISTRY, Bd. 28, Nr. 1, Februar 2000 (2000-02), Seiten 1-15, XP002215565 ISSN: 0045-2068**
• **FRONZA GIOVANNI ET AL: "Stable isotope characterization of raspberry ketone extracted from Taxus baccata and obtained by oxidation of the accompanying alcohol (Betuligenol)." JOURNAL OF AGRICULTURAL AND FOOD CHEMISTRY, Bd. 47, Nr. 3, März 1999 (1999-03), Seiten 1150-1155, XP002215566 ISSN: 0021-8561 in der Anmeldung erwähnt**
• **FRONZA GIOVANNI ET AL: "Stereochemistry of the Baeyer-Villiger-Type Conversion of 4-(4-Hydroxyphenyl)butan-2-one (Raspberry Ketone) into Tyrosol Mediated by Beauveria bassiana" JOURNAL OF ORGANIC CHEMISTRY, Bd. 61, Nr. 26, 1996, Seiten 9362-9367, XP002215567**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **SCHMID JUERG ET AL: "Molecular organization of the shikimate pathway in higher plants." PHYTOCHEMISTRY (OXFORD), Bd. 39, Nr. 4, 1995, Seiten 737-749, XP002215568 ISSN: 0031-9422**

- **WADA E. ET AL: 'Stable Isotopes in the Atmosphere', 1995, KYOTO UNIVERSITY PRESS, KYOTO, JP**
- **SCHREIER P. ET AL: 'Natural Product Analysis', 1998, SPRINGER, HEIDELBERG, DE**

**Beschreibung**

Die Erfindung betrifft die Bestimmung globaler und positioneller Sauerstoff-Isotopengehalte von organischen Verbindungen zum Zwecke der Erkennung ihrer Herkunft und Synthese auf der Basis wissenschaftlicher Erkenntnisse, Interpretationen oder Voraussagen, abgeleitet aus den Mechanismen entsprechender (Bio)synthesen.

Wie alle Haupt-Bioelemente kommen Sauerstoff und Wasserstoff in der Natur als Gemische stabiler Isotope mit den "Relativen Mittleren Häufigkeiten" $^{16}O$ = 99.6337 Atom-%, $^{17}O$ = 0.0374 Atom-% und $^{18}O$ = 0.2039 Atom-% bzw. $^{1}H$ = 99,9822 Atom-%, $^{2}H$ = 0,0145 Atom-% vor. Zur Charakterisierung des Isotopengehalts bestimmter Substanzen wird meistens das Isotopenverhältnis R (ratio) benutzt, der Quotient der relativen Häufigkeit eines schweren Isotops zu der des Haupt-Isotops (für $^{18}O$ z.B. im Mittel R = 0.0020439 für $^{2}H$ 0.000145). Schließlich werden genaue Angaben örtlicher und zeitlicher Abweichungen vom Mittelwert als relative Differenzen zum Isotopenverhältnis eines Internationalen Standards in sog. δ-Werten angegeben. Der Standard für $^{18}O$ und $^{2}H$ ist das "Vienna Standard Mean Ocean Water" (V-SMOW) der IAEA in Wien mit R = 0.0020052 bzw. 0.00015576. Hierauf bezogen ergibt sich für eine beliebige Probe z.B. der

$$\delta^{18}O\text{-Wert }[‰]_{V\text{-SMOW}} = [R_{Probe}/R_{V\text{-SMOW}} - 1] \times 1000$$

Für die Bestimmung des globalen Sauerstoff-Isotopenverhältnisses einer Probe wird sie unter Ausschluss von $O_2$ in einem Vakuumsystem durch Pyrolyse zersetzt, wobei der organisch gebundene Sauerstoff in CO übergeführt wird; $H_2O$ wird mit $CO_2$ isotopisch äquilibriert. Die Messgase werden in einem Isotopenverhältnis-Massenspektrometer, automatisch im Vergleich zu dem Standard, auf das Verhältnis der entsprechenden isotopologen Moleküle analysiert, z. B. bei CO auf das der Massen $^{12}C^{16}O$ (M = 28) und $^{12}C^{18}O$ (M = 30). Häufig wird die Pyrolyse-Isotopenverhältnis-Massenspekrometrie (P-IRMS) mit der gaschromatographischen Auftrennung der zu untersuchenden Verbindungen gekoppelt (GC-P-IRMS). Die benötigte Substanzmenge liegt bei ca. 0.1 mg/Verbindung, die Fehlerbreite der Messung einschließlich Proben-Aufbereitung bei 0.3 ‰.

Positionelle Sauerstoff-Isotopenanalysen (Bestimmung von Isotopenmustern) erfordern den gezielten Abbau der organischen Verbindungen durch geeignete chemische Verfahren und die Isotopenverhältnisbestimmung an den Fragmenten; hierzu werden jeweils ca. 20 mg Probe benötigt.

Die.Bestimmung von Deuterium-($^{2}H$)-Mustern organischer Naturstoffe durch Kernmagnetische Resonanz-Spektrometrie (NMR) ist zum Nachweis der Zuckerung von Wein und Obstsäften [Martin und Martin, 1988; Martin, 1992] und zur Erkennung der Herkunft und Authentizität von Aroma- und Duftstoffen [Martin et al., 1990; Hanneguelle et al., 1992] seit langem Routine; (FR-A-2517828; EP-A-0090901; US-A-4550082). Herkunfts-Zuordnungen und Authentizitäts-Untersuchungen an unbekannten Proben mittels dieser Methode werden bisher nahezu ausschließlich allein auf empirischer Basis durchgeführt, indem deren Daten mit jenen von authentischem Referenzmaterial verglichen werden. Einziges Beispiel der kausalen Interpretation eines (allerdings massenspektrometrisch bestimmten partiellen) Deuterium-Musters ist der von uns beschriebene Zusammenhang der relativen $^{2}H$-Häufigkeit an der Carbonylgruppe von Benzaldehyd (Bittermandelöl) und dessen Synthese (Butzenlechner et al., 1988).

Die Isotopenverhältnisse aller Bioelemente erleiden bei der Synthese organischer Verbindungen aufgrund von thermodynamischen bzw. kinetischen Isotopeneffekten, d.h. dem verschiedenen Verhalten isotopologer bzw. isotopomerer Moleküle bei Phasenübergängen bzw. (bio)chemischen Reaktionen, geringfügige Verschiebungen (d.h. Änderungen der Verhältnisse dieser Moleküle in einer gegebenen Population). So haben z.B. die verschiedenen Spezies von Wasser ($^{1}H^{1}H^{16}O$, $^{1}H^{2}H^{16}O$, $^{1}H^{1}H^{18}O$ usw.) verschiedene Dampfdrücke, was bei Verdampfungen und Kondensationen zu Verschiebungen ihrer Verhältnisse zwischen Vorlage und Kondensat führt.

Dementsprechend werden der Wasserstoff- und der Sauerstoff-Isotopengehalt des Blattwassers von Pflanzen, damit auch die Isotopengehalte der von diesen synthetisierten Naturstoffe, primär durch zeitliche und örtliche klimatische Faktoren (Niederschlag, Verdunstung) bestimmt, weiterhin durch charakteristische Isotopendiskriminierungen im Verlaufe der Biosynthese aufgrund der Mechanismen und Isotopeneffekte der daran beteiligten Reaktionen. Umgekehrt erlaubt die Analyse der globalen und positionellen Isotopengehalte von Naturstoffen, Lebensmitteln oder deren Inhaltsstoffen Aussagen über deren geographischen und biologischen Ursprung sowie über die beteiligten Synthese-Reaktionen und deren Umstände.

Im Falle der Kohlenstoff-Isotopenverhältnisse ist die Interpretation und sogar die Voraussage von Daten heute weitgehend wissenschaftlich fundiert; bei Wasserstoff und Sauerstoff beschränkt sie sich auf die o.g. Zusammenhänge mit klimatischen Faktoren, war hierbei aber im Wesentlichen auf empirische Daten angewiesen, d.h. auf den Vergleich mit authentischen Standardsubstanzen und der Erstellung von Datenbanken. Verfahren zur systematischen kausalen Interpretationen bzw. Voraussagen von Häufigkeiten oder Mustern aller Bioelemente bei Naturstoffen finden sich in früheren Arbeiten des Erfinders [Schmidt H.-L. et al., 1995; Schmidt H.-L., Gleixner G., 1998], auf denen die vorliegende Erfindung basiert.

Auf empirischer Basis wurden bisher über globale $^{18}$O-Analysen, oft verbunden mit entsprechenden Bestimmungen an weiteren Elementen, folgende praktische Resultate in der Herkunfts- und Authentizitätsbestimmung bei Lebensmitteln und deren Inhaltsstoffen erzielt:

Herkunftsbestimmung von Wein auf der Basis der $^{18}$O-Analyse des Wassers [Rossmann et al., 1999].

Erkennung der Wässerung bzw. Zuckerung von Obstsäften sowie Unterscheidung zwischen Originalsäften und rückverdünnten Fruchtsaft-Konzentraten [Bricout et.al., 1973; Rossmann u. Trimborn, 1996; Houerou et al., 1999].

Bestimmung der Herkunft von Butter (relevant bei Subventionsbetrug) [Rossmann et al., 2000]; Verfahren gilt im Prinzip auch für Milch und andere Milchprodukte sowie für Fleisch.

Bestimmung der Herkunft von Olivenöl [Angerosa et al., 1999] bzw. von Glycerin (Zuordnung zu pflanzlichen, tierischen und synthetischen Quellen, mögliche Bedeutung bei Weinfälschungen [Rossmann et al., 1998]).

Unterscheidung zwischen natürlichen und synthetischen Aromastoffen, z.B. "Furaneol", (Aromastoff aus Erdbeeren), Vanillin.

Für solche Herkunftsbestimmungen auf empirischer Basis wurden stets nur die globalen $\delta^{18}$O-Werte bestimmt, entweder von Wasser selbst oder von Substanzen, deren $\delta^{18}$O-Wert zu dem des bei ihrer Entstehung anwesenden Wassers korreliert. Die Herkunftszuordnung beruht im Wesentlichen darauf, dass der globale $\delta^{18}$O-Wert des Niederschlags- und Grundwassers, damit der von Blatt- und Fruchtwasser, mit der geographischen Lage des Anbaugebiets zusammenhängt. Alle Werte können aber durch örtliche und zeitliche Faktoren beeinflusst werden (Regen vor der Weinlese, andere lokale klimatische Einflüsse); sie können deshalb erheblich überlappen, so dass sie im Falle gerichtsrelevanter Begutachtungen meistens mit anderen Parametern kombiniert werden.
Positionelle $\delta^{18}$O-Werte werden darüber hinaus durch biochemische Faktoren bestimmt. Die einzigen bisher publizierten Resultate positioneller $\delta^{18}$O-Werte [Fronza et al., 1999] enthalten aber keine systematische Interpretation auf der Basis von Biosynthesen.
Ähnliche Betrachtungen können hinsichtlich der $\delta^{2}$H-Werte angestellt werden. Es wird auf die vorstehend erwähnte Messmethodik hingewiesen; [Martin et al., verschiedene Arbeiten].
Die Erfindung wird nachstehend anhand bevorzugter Ausführungsformen auch unter Bezugnahme auf die beigefügten Zeichnungen genauer erläutert.

Figur 1 zeigt Schema 1. Schema 1 erläutert die Sauerstoff-Isotopenkorrelationen von Naturstoffen zu primären Sauerstoff-Quellen und mit dem O-Einbau verbundene Gleichgewichts- (EIE) bzw. kinetische (KIE) Isotopeneffekte. Die Zahlen geben die jeweiligen $\delta^{18}$O-Werte oder ihre Bereiche in [‰]$_{V-SMOW}$ an.

Figur 2 zeigt Schema 2. Schema 2 erläutert die Biosynthese von Phenylalanin und Tyrosin bei Pflanzen und Mikroorganismen. Das O-Atom in Tyrosin stammt hier letztlich aus (Blatt)wasser; es geht unmittelbar auf den Zucker Erythrose zurück, dessen O-Atome aufgrund eines EIE gegenüber $H_2O$ eine Anreicherung um ca. 30 ‰ erfahren. Für Tiere ist Phenylalanin essentiell; sie machen daraus Tyrosin durch Einbau von O aus Luft-$O_2$ ($\delta^{18}$O = + 23.5 [‰]$_{V-SMOW}$). Dabei erfährt das O-Atom aufgrund eines KIE eine Abreicherung um ca. 20 ‰. PEP = Phosphoenolpyruvat.

Figur 3 zeigt Schema 3. Schema 3 erläutert die Umarbeitung von Tyrosin in 3-(p-Methoxyphenyl)-1-chlorpropan für die $^{18}$O -Analyse der p-Position. $Me_2SO_4$ = Dimethylsulfat; THF = Tetrahydrofuran; $Ph_3P$ = Triphenylphosphin, EtOH = Ethanol.

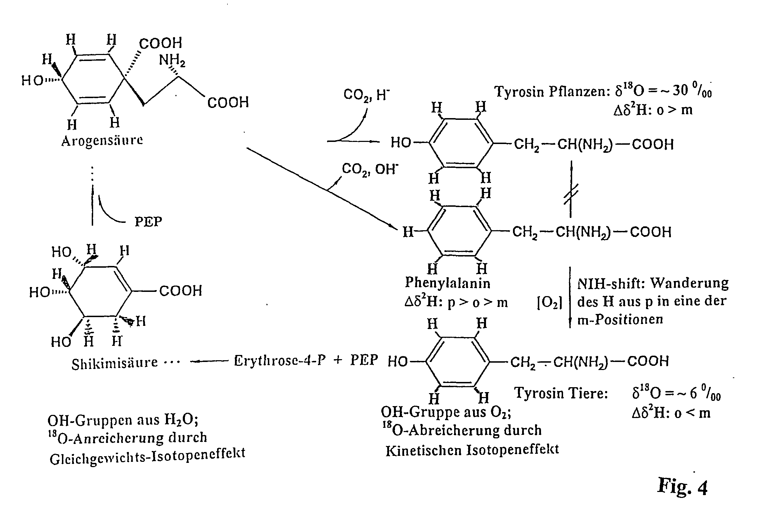

Figur 4 zeigt Schema 4. Schema 4 zeigt die Herkunft von $\delta^{18}$O-Werten phenolischer OH-Gruppen und relative $^2$H-Muster in Tyrosin pflanzlicher und tierischer Herkunft.

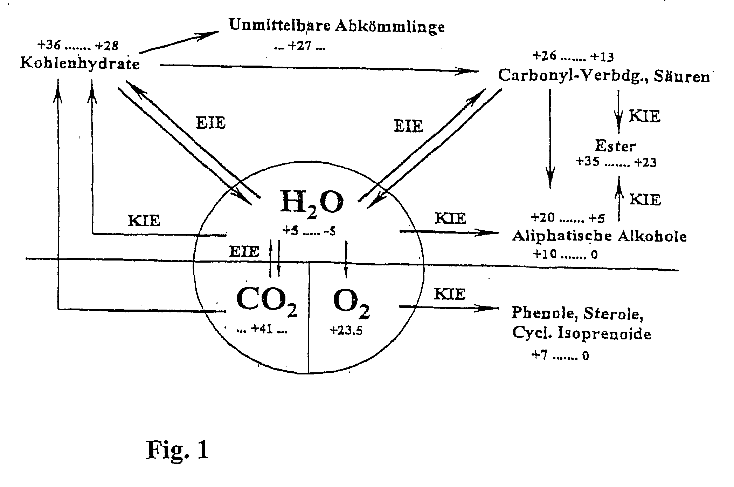

Die vorliegende Erfindung basiert auf von den Erfindern erkannten biosynthetischen Zusammenhängen, Interpretationen und Voraussagen von Sauerstoff-Isotopengehalten und -Isotopenmustern aller denkbaren Naturstoffe (und ihrer synthetischen Analoga), von denen die (Bio)synthese bekannt ist. Auf der Basis der Isotopengehalte der Primärstoffe für den Sauerstoff in organischen Verbindungen ($CO_2$, $H_2O$, $O_2$), der Mechanismen ihrer (Bio)synthesen und der damit verbundenen bekannten und erwarteten Isotopeneffekte (s. Schema, nach Schmidt et al., 1995) können demnach heute $\delta^{18}$O-Werte beliebiger Naturstoffe vorausgesagt werden; alle bisher verfügbaren experimentellen Daten bestätigen entsprechende Voraussagen. Umgekehrt kann aus dem globalen $\delta^{18}$O-Wert eines Naturstoffs mit vielen Sauerstoffatomen aufgrund der von den Erfindern gefundenen Korrelationen zum Blattwasser der Pflanzen dessen $\delta^{18}$O-Wert und damit die Herkunft der organischen Verbindung.abgeleitet werden, vgl. Fig. 1 (Schema 1).

Auf der Basis von bekannten (Bio)synthesen, ihrer Mechanismen, den Reaktionsbedingungen und Isotopeneffekten sollen $^{18}$O-Isotopenhäufigkeiten und -muster von Naturstoffen und entsprechenden synthetischen Analogen abgeleitet werden. So sollen experimentelle Daten unbekannter Proben eine Interpretation ihrer Herkunft und Authentizität erfahren. Insbesondere ist zunächst an die Unterscheidung von L-Tyrosin pflanzlicher bzw. tierischer Herkunft als Basis für die Erkennung der Verwendung tierischer Eiweißquellen bei der Fütterung von Wiederkäuern gedacht.

Erfindungsgemäß wird die positionelle Bestimmung des $\delta^{18}$O-Wertes von L-Tyrosin zur Erkennung der Verfütterung von Tiermehl an Wiederkäuer, also zum Nachweis der Umgehung des entsprechenden Verbotes vorgeschlagen. Am $\delta^{18}$O-Wert der p-Hydroxygruppen muss sich Tyrosin aus rein tierischer Biosynthese (aus Phenylalanin) [theoretischer $\delta^{18}$O-Wert $5 \pm 2$ ‰] von dem aus pflanzlichen Quellen [theoretischer Wert $30 \pm 3$ ‰] charakteristisch und eindeutig unterscheiden; erste experimentelle Daten bestätigen voll die Voraussage (s. nachstehende Tabelle).

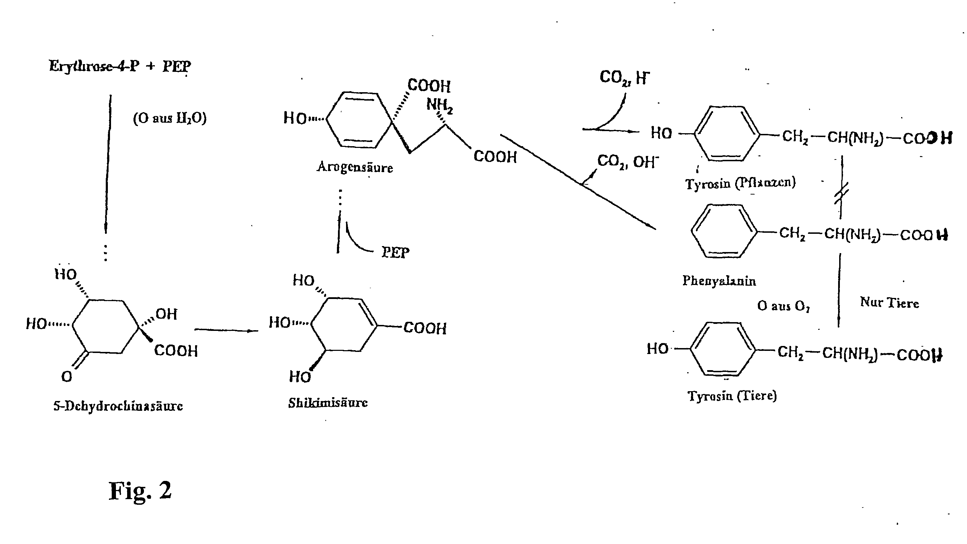

Die Aminosäure Tyrosin (Tyr) ist für höhere Tieren nicht essentiell; sie wird dort durch einen einzigen Reaktionsschritt (Hydroxylierung) aus der essentiellen Aminosäure Phenylalanin (Phe) hergestellt. Pflanzen und Mikroorganismen stellen beide Aminosäuren jeweils aus dem gleichen Vorprodukt Arogensäure (bzw. Prephensäure) dar; eine Hydroxylierung von Phe zu Tyr wird hier fast nie beobachtet [Haslam 1993], vgl. Fig. 2 (Schema 2).

Entsprechend Schema 2 entstammt somit das Sauerstoffatom in p-Position von Tyr aus pflanzlicher Produktion dem Vorläufer Arogensäure und damit dem Primärprodukt Erythrose (Zucker); es geht letztlich auf $H_2O$ (unter $^{18}$O-Anreicherung durch einen Isotopeneffekt) zurück. Bei Tyr aus tierischer Synthese rührt es aus $O_2$ der Luft her, von wo es unter $^{18}$O-Abreicherung (Isotopeneffekt erstmalig von uns postuliert) entsprechend Schema 2 eingebaut wird. Der nach Schema 1 zu erwartende $\delta^{18}$O-Wert in dieser Position ist für pflanzliches Tyr ca. $+30 \pm 3$ ‰ (Die Schwankungsbreite ist durch das Grund- bzw. Blattwasser bedingt; sie kann bei dessen Kenntnis "normalisiert", d.h. korrigiert werden), der für von Tieren aus Phe hergestelltes ca. $+5 \pm 2$ ‰.

Isolierung von Tyr aus biologischem Material und $^{18}$O-Analyse in der p-Position.

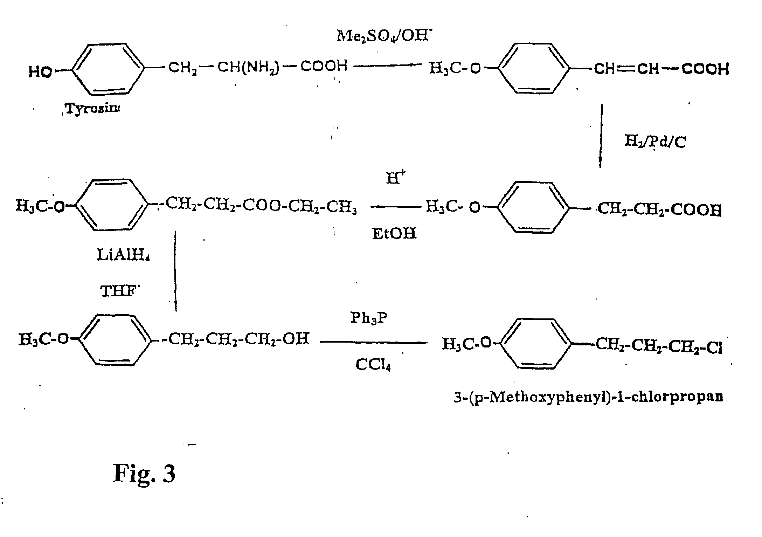

Tyr ist im Casein der Milch mit 6.3, im Muskelprotein von Rindern mit 4.2 Gew.-% enthalten; die Hydrolyse dieser Proteine ist beschrieben, die Isolierung der Aminosäure aus dem Hydrolysat wegen ihrer Schwerlöslichkeit bekannt und relativ unproblematisch. Zur entsprechenden Isolierung aus vernetzten Keratinen (Haare, Hufe, Klauen) müssen entsprechende Verfahren adaptiert werden, Fig. 3 (Schema 3).

Vor der Sauerstoff-Isotopenanalyse in der p-Position müssen die anderen Sauerstoffatome eliminiert werden, da sie die Aussage beeinträchtigen würden. In Anwendung bekannter Methoden der organischen Chemie wurde ein Verfahren entwickelt (Schema 3), das in zufriedenstellender Ausbeute zum Ziel führt (benötigt werden 50 mg Tyr, erhalten werden 10 mg 3-(p-Methoxyphenyl)-1-chlorpropan).

Die mit bekannten Methoden (s. Teil 2) an dieser Verbindung für die p-Position von Tyr verschiedener Herkunft bzw. Tyrosol, einem natürlichen Abkömmling von L-Tyrosin aus Pflanzen, erhaltenen $\delta^{18}$O-Werte waren in [‰]$_{V-SMOW}$ (Tyrosol normalisiert auf $\delta^{18}$O für Blattwasser = 0 ‰)

Tabelle

| | | Tyrosol | +30,2 |
|---|---|---|---|
| | | Tyr aus Casein | +23,5 |
| Tyr kommerziell (Aldrich) | +31,5 | Tyr aus Hühnerfedern | +18,9 |
| | | Tyr aus menschl. Haar | +17,8 |
| | | Vanillin (als Modell für eine p-hydroxylierte Verbindung) | + 5,8 |

Die Quellen für das kommerzielle Tyr waren nicht bekannt, doch ist anzunehmen, dass es aus pflanzlichem Protein isoliert wurde. Die Probe aus Casein entspricht vollständig den Erwartungen für ein Tyr, das überwiegend auf pflanzliche und/oder mikrobielle Biosynthese und partielle Eigensynthese zurückgeht. Der Anteil an "pflanzlichem Tyrosin" ist 70%. Demgegenüber sind die Proben von "Gemischkost-Ernährten" im Einklang mit einem Ursprung aus pflanzlichen und tierischen Quellen (einschließlich der Eigenproduktion aus Phe); aus den oben angegebenen erwarteten Werten berechnet sich für das menschliche Haar der Anteil von pflanzlichem Tyr zu 47 %.

Das Resultat beweist beispielhaft, dass die Vorhersage von positionellen und globalen Sauerstoff-Isotopenhäufigkeiten aus den Mechanismen der infrage kommenden Synthesen möglich ist. Im vorliegenden Fall ergibt sich daraus die Möglichkeit, an einer Milchprobe den Nachweis einer (verbotenen) Fütterung mit Tiermehl zu führen; es wird darüber hinaus möglich sein, den Beweis einer solchen Fütterung rückwirkend an Fleisch, Haaren oder Hörnern zu führen.

**Literaturverzeichnis**

Angerosa F., Bréas O., Contento S., Guillou C., Reniero F., Sada E. [1999]: Application of stable isotope ratio analysis to the characterisation of the geographical origin of olive oils. J. Agric. Food Chem. 47, 1013 - 1017

Bricout J., Merliot L. Fontes J.-C. [1973] : Sur la composition en isotopes stables de l'eau de jus de pommes. Indust. Aliment. Agric. 90(1), 19 - 22

Fronza G., Fuganti C., Pedrocchi-Fantoni G., Serra S., Zucchi G., Fauhl C., Guillou C., Reniero F. [1999]: Stable isotope characterisation of raspberry ketone extracted from *Taxus baccata* and obtained by oxidation of the accompanying alcohol (betuligenol). J. Agric. Food Chem. 47, 1150 - 1155

Haslam E. [1993]: Shikimic Acid, Metabolism and Metabolites, John Wiley & Sons, Chichester

Houerou G., Kelly S.D., Dennis M.J. [1999]: Determination of the oxygen-18/oxygen-16 isotope ratios of sugar, citric acid and water from single strength orange juice. Rapid Commu-nications in Mass Spectrometry 13, 1257 - 1262

Martin, G.J., Martin, M.L. (1988): The site-specific natural isotope fractionation-NMR method applied to the study of wines. In Linskens, H.F., Jackson, J.F. (eds.): Modem Methods of Plant Analysis, Vol. 6. Springer Verlag, pp 258- 275.

Martin, G.J., Hanneguelle, S., Remaud, G. (1990): Authentification des aromes et parfums par resonance magnétique nucléaire et spectrométrie de masse de rapports isotopiques. Parfums Cosmét. Arômes 94, 95-109.

Butzenlechner, M., Rossmann, A., Schmidt, H.-L. [1988]. Assignment of bitter almond oil to natural and synthetic sources by stable isotope analyses. J. Agric. Food Chem. 37, 410 -412

Hanneguelle, S., Thibault, J.-N., Naulet, N., Martin, G.J. [1992]. Authentication of essential oils containing linalool and linalyl acetate by isotopic methods. J. Agric. Food Chem. 40, 81-87.

Martin, G. (1992): SNIF-NMR - eine Methode zum Nachweis von Rübenzuckerzusatz in Fruchtsäften. Flüssiges Obst .59, S. 477 - 485.

Rossmann A. Trimbom P.[1996]: Gehalte von stabilen Sauerstoff-Isotopen im Wasser von Apfelsaft-Konzentrat als Kriterium für den Nachweis einer Zuckerung. Z Lebensm.Unters. Forsch 203, 277 - 282

Roßmann A., Schmidt, H.-L., Hermann A., Ristow R. [1998]: Multielement stable isotope ratio analysis of glycerol to determine its origin. Z. Lebensm.-Unters. Forsch. A 207, 237-243.

Roßmann A., Reniero F., Moussa I., Schmidt H.-L. Versini G., Merle M.H. [1999]: Stable oxygen content of water of EU data bank wines from Italy, France and Germany. Z. Lebensm.-Unters. Forsch. A 208, 169 - 176

Roßmann A., Haberhauer G., Hölzl S., Horn P., Pichlmayer F. Voerkelius S. [2000]: The potential of multielement stable isotope analysis for the regional origin assignment of butter. Eur. Food Res. Technol. 211, 32 - 40

Schmidt H.-L., Kexel H., Butzenlechner M., Schwarz S., Gleixner G., Thimet S., Werner R.A., Gensler M. [1995]. Non-statistical isotope distributions in natural compounds: Mirror of their biosynthesis and key for their origin assignment. In Wada E., Yoneyama T., Minagawa M., Ando T., Fry B.D. (Eds.), Stable Isotopes in the Biosphere. Kyoto University Press, Kyoto, 17 - 35, ISBN: 4876980225.

Schmidt H.-L., Gleixner G. [1998]. Isotopic patterns in natural compounds. Origin and importance in authenticity analysis. In Schreier P., Herderich M., Humpf H.-U., Schwab W. (Eds.). Natural Product Analysis, Chromatography - Spectroscopy - Biological Testing. Springer, Heidelberg, 271-280, ISBN: 3540670106.

**Patentansprüche**

1. Verfahren zur Ermittlung der Herkunft biologisch, biochemisch und ernährungsphysiologisch relevanter Stoffe unter Ausnutzung ihrer biosynthetisch voraussagbaren Isotopenmuster, wobei die Bestimmung des aus der Biosynthese zu erwartenden Wertes anhand der Kenntnis der Mechanismen der möglichen Synthesewege vorhergesagt wird und darauf aufbauend deren Unterscheidung von synthetischen Analoga aufgrund ihrer relativen positionellen Sauerstoff-Isotopenverhältnisse ($\delta^{18}$O-Wert), indem man in den zu untersuchenden Stoffen die positionellen $\delta^{18}$O-Werte einzeln bestimmt und sie mit den aus der Biosynthese erwarteten vergleicht, wobei eine Übereinstimmung oder Abweichung die Authentizität bzw. Herkunft oder Fälschung des Stoffes zu erkennen erlaubt, **dadurch gekennzeichnet, dass** der biologisch, biochemisch und ernährungsphysiologisch relevante Stoff L-Tyrosin aus tierischen Proteinen, z.B. Casein ist, das positionelle phenolische Sauerstoffatom ($\delta^{18}$O-Wert) in Betracht gezogen wird, und ein Vergleich der Werte des positionellen phenolischen Sauerstoffatoms von L-Tyrosin die Angabe der Herkunft der metabolischen Vorstufen des zu untersuchenden tierischen Proteins, z.B. Caseins erlaubt.

2. Verfahren nach Anspruch 1 , indem man das L-Tyrosin in die p-Methoxyzimtsäure umwandelt, diese hydriert, verestert, die Estergruppe hydrierend spaltet und mit Triphenylphosphin und $CCl_4$ zu 3-(p-Methoxyphenyl)-1-chlorpropan umsetzt, wobei dieses zur Messung des Sauerstoff-Isotopenverhältnisses des positionellen Sauerstoffs im L-Tyrosin herangezogen wird.

3. Verfahren nach Anspruch 1, wobei in biologischem Material die Herkunft des organisch gebundenen Sauerstoffs aus Blattwasser ($\delta^{18}$O-Wert +5........-5 ‰$_{V\text{-}SMOW}$, 2 - 8 ‰$_{V\text{-}SMOW}$ über dem des jeweiligen Grundwassers) bzw. aus Luft-$O_2$ ($\delta^{18}$O-Wert +23.5 ‰$_{V\text{-}SMOW}$) ist, deren O-Atome beim Einbau eine charakteristische Isotopendiskriminierung erfahren.

**Claims**

1. Process for determining the origin of substances with biological, biochemical and nutritional physiological relevance by utilising their biosynthetically predictable isotope pattern, whereby the determination of the value to be expected from biosynthesis is predicted on the basis of knowledge of the mechanisms of the possible synthetic pathways and the resulting differentiation of synthetic analogues based on their relative positional oxygen isotope ratio ($\delta^{18}$O value) in that the positional $\delta^{18}$O values or are determined individually in the substance to be examined and are compared with the values expected from biosynthesis, whereby agreement or difference allows the authenticity/ source or faking of the substance to be recognised, **characterised in that** the biologically, biochemically and nutritional physiologically relevant substance L-tyrosine is from animal proteins, e.g. casein, that the positional phenolic oxygen atom ($\delta^{18}$O value) is taken into consideration, and a comparison of the values for the positional phenolic oxygen atom of L-tyrosine allows the origin of the metabolic precursors of the animal protein being examined, e.g. casein, to be stated.

2. A process according to Claim 1, whereby L-tyrosine is converted to p-methoxycinnamic acid, which is hydrogenated, esterified, hydrogenate to cleave the ester group and reacted with tripehnylphosphine and $CCl_4$ to give 3-(p-methoxyphenyl)-1-chlorpropane, whereby this is used to measure the oxygen isotope ratio of the positional oxygen in the L-tyrosine.

3. A process according to Claim 1, whereby the source of the organically bound oxygen in biological material is either from leaf water ($\delta^{18}$O value +5 ........5‰ $_{V\text{-}SMOW}$, 2-8‰$_{V\text{-}SMOW}$ through the relevant ground water) or from atmospheric $O_2$ ($\delta^{18}$O value +23.5% $_{V\text{-}SMOW}$), the O atoms of which undergo characteristic isotope discrimination during incorporation.

**Revendications**

1. Procédé pour déterminer l'origine de produits pertinents du point de vue biologique, biochimique et physiologique nutritionnel en utilisant leur modèle d'isotope prévisible de manière biosynthétique, la détermination de la valeur attendue de la biosynthèse étant prévue sur la base des connaissances des voies de synthèse possibles et à partir de ces données, leur distinction des analogues de synthèse sur la base de leur rapport positionnel relatif de l'oxygène à l'isotope (valeur $\delta^{18}$O), en déterminant individuellement dans les substances à analyser, les valeurs positionnelles de $\delta^{18}$O et en les comparant à celles attendues de la biosynthèse, une correspondance ou un écart permettant de déterminer l'authenticité ou l'origine ou la falsification de la substance, **caractérisé en ce que** la substance L-tyrosine pertinente du point de vue biologique, biochimique et physiologique nutritionnel provient de protéines animales, par exemple la caséine, l'atome d'oxygène phénolique positionnel (valeur $\delta^{18}O$) étant pris en considération et une comparaison des valeurs de l'atome d'oxygène phénolique positionnel de L-tyrosine permettant d'indiquer l'origine des étapes métaboliques précédentes de la protéine animale à analyser, par exemple la caséine.

2. Procédé selon la revendication 1, **caractérisé en ce que** l'on transforme la L-tyrosine en acide p-méthoxy-cinnamique, on hydrogène celui-ci, on l'estérise, le groupe ester étant clivé de manière hydrogénante et transformé avec la triphénylphosphine et le $CCl_4$ en 3-(p-méthoxyphényl)-1-chloropropène, celui-ci étant utilisé pour la mesure des rapports de l'oxygène à l'isotope de l'oxygène positionnel dans la L-tyrosine.

3. Procédé selon la revendication 1, dans lequel dans le matériau biologique, l'origine de l'oxygène fixé de manière organique provient de l'eau des feuilles (valeur $\delta^{18}O$ + 5...-5 ‰ V-SMOW, 2 - 8 V-SMOW supérieur à celui de l'eau souterraine respective) ou de l'$O_2$ de l'air (valeur $\delta^{18}O$ +23,5 ‰ V-SMOW), dont les atomes d'oxygène subissent lors de l'incorporation, une discrimination isotopique caractéristique.

Fig. 1

Erythrose-4-P + PEP

(O aus H₂O)

COOH
NH₂
HO‴ Arogensäure
COOH

CO₂, H⁺

HO— —CH₂—CH(NH₂)—COOH
Tyrosin (Pflanzen)

CO₂, OH⁻

—CH₂—CH(NH₂)—COOH
Phenylalanin

O aus O₂    Nur Tiere

HO— —CH₂—CH(NH₂)—COOH
Tyrosin (Tiere)

PEP

HO
HO‴
HO‴ OH
COOH
O
5-Dehydrochinasäure

HO
HO‴
HO‴ COOH
HO
Shikimisäure

**Fig. 2**

EP 1 346 216 B1

Me$_2$SO$_4$/OH$^-$

HO—⟨phenyl⟩—CH$_2$—CH(NH$_2$)—COOH ⟶ H$_3$C-O—⟨phenyl⟩—CH=CH—COOH

Tyrosin

H$_2$/Pd/C

H$_3$C-O—⟨phenyl⟩—CH$_2$-CH$_2$-COO-CH$_2$-CH$_3$ ⟵ H$_3$C-O—⟨phenyl⟩—CH$_2$-CH$_2$-COOH

H$^+$

EtOH

LiAlH$_4$

THF

H$_3$C-O—⟨phenyl⟩—CH$_2$-CH$_2$-CH$_2$-OH ⟶ H$_3$C-O—⟨phenyl⟩—CH$_2$-CH$_2$-CH$_2$-Cl

Ph$_3$P

CCl$_4$

3-(p-Methoxyphenyl)-1-chlorpropan

## Fig. 3

11

Fig. 4